(19) Europäisches Patentamt
European Patent Office
Office européen des brevets

(11) **EP 2 716 767 A1**

(12) **EUROPEAN PATENT APPLICATION**

(43) Date of publication:
**09.04.2014 Bulletin 2014/15**

(51) Int Cl.:
***C12Q 1/68*** *(2006.01)*

(21) Application number: **12306209.3**

(22) Date of filing: **04.10.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **SKULDTECH**
**34790 Grabels (FR)**
• **AB Science**
**75008 Paris (FR)**

(72) Inventors:
• **Piquemal, David**
**30380 Saint Christol les Ales (FR)**
• **Moussy, Alain**
**75006 Paris (FR)**

• **Kinet, Jean-Pierre**
**Lexington, MA Massachusetts 02421 (US)**
• **Montestruc, François**
**92240 Malakoff (FR)**

(74) Representative: **Portal, Frédéric**
**Cabinet Lavoix**
**2, Place d'Estienne d'Orves**
**75441 Paris Cedex 09 (FR)**

Remarks:
A request for correction of description has been filed pursuant to Rule 139 EPC. A decision on the request will be taken during the proceedings before the Examining Division (Guidelines for Examination in the EPO, A-V, 3.).

(54) **Method for determining the prognosis of pancreatic cancer**

(57)     The present invention relates to an *in vitro* method for determining the prognosis of pancreatic cancer in a patient. More particularly, this method comprises the following steps:
c) measuring the expression level of at least two genes chosen in the group consisting in: ING5, RUNDC1, ABCC1, FGFR3, S100A6, ANKRD55, and CD8A or homologous genes, in a blood sample of said patient,
d) predicting the outcome of the pancreatic cancer in said patient.

The invention also relates to a kit specifically designed to carry out such a method.

EP 2 716 767 A1

**Description**

The present invention relates to pancreatic cancer and more particularly to the prognosis of pancreatic cancer, especially of a pancreatic cancer treatment.

With 43,920 new diagnoses in the United States each year, and 37,390 deaths, mortality is over 85%, making pancreatic cancer the fourth highest cancer killer in the United States amongst both men and women.

The incidence of pancreatic cancer has markedly increased over the past several decades. Each year about 60,000 individuals in Europe, and more than 230,000 worldwide, are diagnosed with this condition.

Patients diagnosed with pancreatic cancer have often a poorer prognosis compared to other malignancies, in part because early detection is difficult. At the time of diagnosis, most patients with pancreatic ductal adenocarcinoma present with locally advanced or metastatic disease, and only 10-20% of cases are candidates for curative surgery. Median survival from diagnosis is around 3 to 6 months; 5-year survival is much less than 5% and complete remission is extremely rare.

Current therapies approved or used in clinical practice in pancreatic cancer patients are gemcitabine, folfirinox and erlotinib.

Gemcitabine is a nucleoside analog, often used in pancreatic cancer treatment. With gemcitabine, the median overall survival varies between 4.9 months and 8.3 months.

Folfirinox is a tritherapy that has shown to increase median overall survival to 11.1 months in a recent phase III study. However, after 2 years, no benefit in survival rates was detectable with folfirinox compared to treatment with gemcitabine alone. Furthermore, the additional toxicity related to folfirinox has negative impact on the treatment.

Erlotinib, the first tyrosine kinase inhibitor approved in combination treatment with gemcitabine, shows therapeutic benefit in terms of overall survival (OS) compared to gemcitabine treatment alone.

The limited treatment success and the continuing high mortality rate among pancreatic cancer patients highlight the high unmet medical need for additional therapeutic, well-tolerated products for this indication, ideally targeting different pathways implicated in the disease.

As an example of compounds targeting different pathways, erlotinib targets the human epidermal growth factor receptor type 1 (HER1 or EGFR), while other tyrorisine kinase inhibitors, such as Masitinib, potently and selectively inhibit the c-Kit wild-type (WT) receptor and several mutant forms of the same receptor.

The treatment of pancreatic cancer with different compounds may have different degrees of efficacy depending on the patient. However, up to today, there has been no means to predict the clinical benefit of the various available treatments. There is, thus, still a need for such prognosis tests in order to select the right treatment, so as to give the best chance to each patient. Said prognosis should be, in particular, a routinely performed test, such as a non-invasive test.

The inventors have identified a set of genes which can predict the outcome in pancreatic cancer, in particular, when a gemcitabine-based treatment is administered to the patient suffering from a pancreatic cancer. Said set of genes can be assessed directly from a blood sample.

The invention thus relates to an *in vitro* method for determining the prognosis of a pancreatic cancer in a patient, comprising the following steps:

> a) Measuring the expression level of at least two genes chosen in the group consisting in ING5, RUNDC1, ABCC1, FGFR3, S100A6, ANKRD55 and CD8A or homologous genes, in a blood sample of said patient;
> b) Predicting the outcome of the pancreatic cancer in said patient.

The term *"homologous"* is defined as a polynucleotide sequence having a degree of identity of at least 80%, preferably 85%, more preferably 90%, and even more preferably 99% of the gene sequence (full length). The degree of identity refers to sequence identity between two sequences. Identity can be determined by comparing a position in each sequence which may be aligned for purposes of comparison. When an equivalent position in the compared sequences is occupied by the same base, then the molecules are identical at that position. Various alignment algorithms and/or programs may be used for determining the homology of two sequences, including FASTA and BLAST.

The method according to the invention is carried out on a blood sample of a patient, preferably on a whole peripheral blood sample of said patient. Peripheral blood is blood that circulates through the heart, arteries, capillaries and veins. The terms *"whole blood'* are used as opposed to a fraction of blood, obtained through separation of particular components of blood. An example of a blood fraction is peripheral blood mononuclear cells.

The method according to the invention is non-invasive because only a simple and routine blood sample collection is required to carry out the method. This is particularly advantageous since it is very difficult to access tumorous cells in pancreatic tissues for biopsy. Additionally, the sampling (collection, stabilization and transport) is standardized and the use of whole blood is safer than the use of a blood fraction such as peripheral blood mononuclear cells (PBNC), since it avoids handling errors related to the preparation of said fractions (for example FICOLL preparation for PBNC).

In a preferred embodiment, the expression level of at least 3, at least 4, at least 5, at least 6 and, more preferably of the 7 genes, is measured.

By "prognosis", it is meant the outcome of the patient in terms of life expectancy. In the case where the prognosis method involves a patient having or about to have a given pancreatic cancer treatment, the "outcome" results from the efficacy and/or the potential benefit of said given pancreatic cancer treatment, in particular, in terms of life expectancy.

Thus, the prognosis of pancreatic cancer, includes more particularly the prognosis of said cancer when a given pancreatic cancer treatment is administered to the patient. "Pancreatic cancer treatment" more specifically encompasses a gemcitabine-based treatment, more preferably, a treatment based on a combination of gemcitabine with a tyrosine kinase inhibitor, still more preferably, a treatment based on a combination of gemcitabine with masitinib.

Advantageously, the expression level of a gene is compared to a reference value, said value being, preferably, a reference expression level of said gene and, more preferably, the median or the first quartile expression level of said gene observed in patients suffering from a pancreatic cancer.

In particular, a lower expression level of at least two of the above-mentioned genes indicates a long-term survival of the patient.

By "lower expression level", it is meant an expression level that is lower by at least 5%, preferably 10%, than the mean expression level observed in patients suffering from a pancreatic cancer.

By "long-term survival", it is understood survival for more than 10 months, preferably more than 12 months, even more preferably more than 15 months.

More precisely, a lower expression level of at least one combination of genes selecting in the group consisting in:

- ING5 and RUNDC1,
- ABCC1 and FGFR3,
- ING5 and S100A6,
- ANKRD55 and CD8A.

indicates a long-term survival of the patient.

By "lower expression level of one combination of genes", it is intended that both genes of the combination have a lower expression level.

A contrario, a higher expression level of all of the above-mentioned genes indicate a short-term survival of the patient.

By "higher expression level", it is meant an expression that is higher by at least 5%, preferably 10%, than the mean expression level observed in patients suffering from a pancreatic cancer.

By "short-term survival", it is meant a survival of less than 6 months, less than 5 months, or less than 3 months.

In a first embodiment, the expression level of a gene is measured as the level of the protein of said gene. In that case, the level of the protein is preferably measured by employing antibody-based detection methods such as immunochemistry or western-blot analysis.

In another embodiment, the expression level of a gene is measured as the level of the RNA transcript or the cDNA of said genes. In that case, the level of RNA transcript(s) or the cDNA is measured by employing nucleic acid based detection methods such as microarrays, quantitative PCR, DNA chips, hybridization with labeled probes, or lateral flow immunoassays, in particular lateral flow dipstick tests.

Preferably, in the method according to the invention, the expression level of the gene is measured by real time quantitative PCR (real time quantitative polymerase chain reaction or qPCR) performed on the RNA transcript or the cDNA of said gene.

A real time quantitative PCR is a PCR wherein the amplified DNA is detected as the reaction progresses in real time. This detection is made through the accumulation of a fluorescent signal. The Ct (cycle threshold) is defined as the number of PCR cycles required for the fluorescent signal to cross the threshold (i.e. exceed background level).

Thus, a forward and a reverse primer, and a reporter, preferably a DNA fluorescent intercalant, are used in a qPCR. Advantageously, primers which are specific for hybridizing within the gene flanking regions are used.

In the case of the ING5 gene, the flanking regions are located on exon on chromosome 2 between nucleotides 242,668,763 and nucleotides 242,668,856 (Assembly Feb. 2009 GRch37/hg19, UCSC source).

In the case of the RUNDC1 gene, the flanking regions are located on exon on chromosome 17 between nucleotides 41,145,524 and nucleotides 41,145,646 (Assembly Feb. 2009 GRch37/hg19, UCSC source).

In the case of the ABCC1 gene, the flanking regions are located on exons on chromosome 16 between nucleotides 16,177,368 and nucleotides 16,180,772 (Assembly Feb. 2009 GRch37/hg19, UCSC source).

In the case of the FGFR3 gene, the flanking regions are located on exon on chromosome 4 between nucleotides 1,809,275 and nucleotides 1,809,378 (Assembly Feb. 2009 GRch37/hg19, UCSC source).

In the case of the S100A6 gene, the flanking regions are located on exon on chromosome 1 between nucleotides 153,507,703 and nucleotides 153507823 (Assembly Feb. 2009 GRch37/hg19, UCSC source).

In the case of the ANKRD55gene, the flanking regions are located on exon on chromosome 17 between nucleotides 41,145,524 and nucleotides 41,145,646 (Assembly Feb. 2009 GRch37/hg19, UCSC source).

In the case of the CD8A gene, the flanking regions are located on exon on chromosome 2 between nucleotides 87,011,736 and nucleotides 87,011,806 (Assembly Feb. 2009 GRch37/hg19, UCSC source).

In a preferred embodiment, the following primers can be used to perform the real time quantitative PCR:

- ING5

  o primer forward: TCCCTTTGTAAAGGAAAGATAATG
  o primer reverse: TCCATAATCTATAACATGATCCAAAAA

- S100A6

  o primer forward: CCTCAGCCATGGC(LNA-base)AC(LNA-base)G
  o primer reverse: ATCAGCTCCTTCAGCTCCTTC

LNA® (Locked Nucleic Acid)® is a nucleic acid analog that contains a 2'-O, 4'-C methylene bridge. The bridge increases the thermal stability, reduces the flexibility and increases the hybridation interactions.

- RUNDC1

  o primer forward: TCACCTTCCCTGTGAAACAA
  o primer reverse: CCAAAGCTGCAGATCTCTAAGG

- ANKRD5

  o primer forward: TCACCTTCCCTGTGAAACAA
  o primer reverse: CCAAAGCTGCAGATCTCTAAGG

- ABCC1

  o primer forward: CCAGTGGGGATCGGACAGA
  o primer reverse: AGGGGATCATCGAAGAGGTAAAT

- CD8A

  o primer forward: GGGTTGTACTATGCTGTTATGAGTG
  o primer reverse: TGTTTACCCATTAAAGAGGATGC

- FGFR3

  o primer forward: ACCCAGTGCAGAATGTAAGTG
  o primer reverse: GTGATGTCCCGTGGTGCAA

The real time quantitative PCR allows one to determine the cycle threshold (Ct) value of gene, said value being normalized with respect to the expression level of a housekeeping gene to give a ΔCt value.

Housekeeping genes are genes that are expressed in all the cells of an organism under normal and pathophysiological conditions. These genes are usually expressed at relatively constant levels. Preferably, the normalization, in the method according to the invention, is based on the expression level of two housekeeping genes, in particular, based on the expression level of genes B2M and GAPDH.

In the case of the B2M gene, the flanking regions are located on exon on chromosome 15 between nucleotides 45,010,919 and nucleotides 45,010,990 (Assembly Feb. 2009 GRch37/hg19, UCSC source).

In the case of the GAPDH gene, the flanking regions are located on exons on chromosome 12 between nucleotides 6,643,999 and nucleotides 6,645,738 (Assembly Feb. 2009 GRch37/hg19, UCSC source).

Primers particularly suitable for the B2M and GAPDH genes can be:

- B2M

  o primer forward: GCTCAGTAAAGACACAACCATCC

o primer reverse: CATCTGTGGATTCAGCAAACC

- GAPDH

o primer forward: ATGGGGAAGGTGAAGGTCG
o primer reverse: GGGGTCATTGATGGCAACAATA

Thus, when two housekeeping genes (for example, genes B2M and GAPDH) are used to normalize the Ct value of a given gene, the ΔCt of said gene is calculated as follows:

$$\Delta Ct = Ct\ (gene) - [Ct\ (B2M) + Ct\ (GAPDH)]/2$$

Advantageously, for performing the real-time quantitative PCR, primers, size (preferably between 80 and 150 nucleotides), Tm (melting temperature, preferably 60°C $\pm$1°C), GC% (percentage of G or C nucleotide, preferably ~60% in 3'), 3' and 5' self-complementarity and stability (preferably inferior to 4 nucleotides), product size ranges and thermodynamic parameters (secondary structure evolution according primer Tm and sodium salt concentration) are selected to allow a simultaneous detection.

According to the method of the invention, a patient presenting at least one of the four following features is predicted to have a long-term survival, and is eligible for a gemcitabine-based treatment, more particularly a gemcitabine + masitinib treatment:

- a ΔCt value for ING5 and RUNDC1 higher than the median ΔCt for said genes,
- a ΔCt value for ABCC1 higher than the median ΔCt for said gene and a ΔCt value for FGFR3 higher than the 1 st quartile ΔCt for said gene,
- a ΔCt value for ING5 and S100A6 higher than the median ΔCt for said genes,
- a ΔCt value for ANKRD55 higher than the median ΔCt for said gene and a ΔCt value for CD8A higher than the 1 st quartile ΔCt for said gene.

By *"median ΔCt"*, it is meant the median of the ΔCt of all of the tested patients, and by "1st quartile ΔCt", it is meant the first quartile of the ΔCt of all of the tested patients. The tested patients are patients suffering from a pancreatic cancer.

These ΔCt values are as follows:

| | |
|---|---|
| - ING5 median ΔCt : | 10.705 |
| - RUNDC1 median ΔCt : | 3.756 |
| - ABCC1 median ΔCt : | 4.109 |
| - FGFR3 1st quartile ΔCt: | 13.082 |
| - S100A6 median ΔCt : | 4.472 |
| - ANKRD55 median ΔCt : | 6.258 |
| - CD8A 1st quartile ΔCt : | 5.303. |

The present invention further relates to a nucleic acid microarray having on its surface nucleic acids consisting of nucleic acids able to hybridize with at least one combination of genes selected in the group consisting of:

- ING5 and RUNDC1
- ABCC1 and FGFR3
- ING5 and S100A6
- ANKRD55 and CD8A,

with optionally nucleic acids specific for at least one housekeeping gene, preferably two housekeeping genes, more preferably for B2M and GAPDH.

The present invention also relates to a kit for determining the prognosis of pancreatic cancer in a patient, comprising means for detecting the level of expression of at least two genes selected from the group consisting in ING5, RUNDC1, ABCC1, FGFR3, S100A6, ANKRD55 and CD8A.

The means for detecting the level of expression can be a microarray according to the invention, a set of primers and a reporter such as fluorescent agents, labeled hydrolysis probes, molecular beacons, hybridization probes, chips and antibodies.

Preferably, the kit according to the invention comprises means for detecting the expression level of a combination of genes selecting in the group consisting in:

- ING5 and RUNDC1,
- ABCC1 and FGFR3,
- ING5 and S100A6,
- ANKRD55 and CD8A.

More preferably, the kit according to the invention comprises means for detecting all the above-mentioned gene combinations.

The kit can further comprise instructions for use in the *in vitro* method according to the invention.

Finally, the invention also concerns the use of at least two genes selected in the group consisting in ING5, RUNDC1, ABCC1, FGFR3, S100A6, ANKRD55 and CD8A for the prognosis of pancreatic cancer, in particular, of a pancreatic cancer treatment.

Preferably, the invention relates to the use of at least one of the combinations of genes selected in the group consisting in:

- ING5 and RUNDC1,
- ABCC1 and FGFR3,
- ING5 and S100A6,
- ANKRD55 and CD8A.
   for said prognosis.

**Example 1: Set of genes for the prognosis of pancreatic cancer**

1. Total blood samples from patients in PAXgene tubes in ice dry (shipper: LabConnect, USA) were received and stored at -80°C.

- Collected tubes belong to 121 patients before treatment, and are named Week 0.
- Total RNA was extracted from the blood samples of 121 patients before treatment, and named week 0. The transcriptome analysis (biomarker investigation) was conducted only on this time point.
- All of the 121 RNA samples were analyzed. If some samples received were not eligible for analysis due to insufficient quality material, they were not used. One sample was eliminated from the study due to poor RNA quality (RNA Integrity Number < 8, see below).
- Digital Gene Expression (DGE) experiments were carried out to select a set of putative biomarkers.
- Biomarker validation was done using Real-Time PCR on COBAS platform (LC480, ROCHE Diagnostics) and appropriate biostatistical approaches has been used to filter best biomarkers.

2. RNA Samples

120 blood RNA samples (Patient ID 4903 was eliminated from the study due to a poor RNA quality), corresponding to baseline blood samples, were extracted from blood (PAXgene Blood collection tubes, BD) using PAXgene Blood RNA Kit V.2 (PreAnalitix) according to manufacturer's recommendations.

| Subject Identifier for the Study | Treatment group | Dead | OS (days) | OS (months) |
|---|---|---|---|---|
| 109 | Masitinib + Gemcitabine | YES | 182 | 6.0 |
| 110 | Placebo + Gemcitabine | YES | 183 | 6.0 |
| 111 | Placebo + Gemcitabine | NO | 744 | 24.4 |
| 112 | Placebo + Gemcitabine | YES | 112 | 3.7 |
| 113 | Placebo + Gemcitabine | NO | 589 | 19.4 |

(continued)

| Subject Identifier for the Study | Treatment group | Dead | OS (days) | OS (months) |
|---|---|---|---|---|
| 207 | Placebo + Gemcitabine | YES | 98 | 3.2 |
| 208 | Placebo + Gemcitabine | YES | 87 | 2.9 |
| 209 | Masitinib + Gemcitabine | YES | 60 | 2.0 |
| 211 | Placebo + Gemcitabine | YES | 160 | 5.3 |
| 506 | Masitinib + Gemcitabine | YES | 147 | 4.8 |
| 507 | Masitinib + Gemcitabine | YES | 92 | 3.0 |
| 508 | Placebo + Gemcitabine | YES | 253 | 8.3 |
| 709 | Masitinib + Gemcitabine | YES | 474 | 15.6 |
| 710 | Masitinib + Gemcitabine | YES | 536 | 17.6 |
| 805 | Placebo + Gemcitabine | YES | 654 | 21.5 |
| 806 | Masitinib + Gemcitabine | YES | 167 | 5.5 |
| 1103 | Masitinib + Gemcitabine | YES | 449 | 14.8 |
| 1104 | Placebo + Gemcitabine | YES | 402 | 13.2 |
| 1203 | Placebo + Gemcitabine | YES | 252 | 8.3 |
| 1204 | Masitinib + Gemcitabine | YES | 436 | 14.3 |
| 1408 | Masitinib + Gemcitabine | YES | 432 | 14.2 |
| 1409 | Masitinib + Gemcitabine | YES | 49 | 1.6 |
| 1501 | Masitinib + Gemcitabine | YES | 47 | 1.5 |
| 1502 | Masitinib + Gemcitabine | YES | 560 | 18.4 |
| 1503 | Masitinib + Gemcitabine | YES | 519 | 17.1 |
| 1609 | Masitinib + Gemcitabine | YES | 498 | 16.4 |
| 1610 | Masitinib + Gemcitabine | YES | 492 | 16.2 |
| 1611 | Masitinib + Gemcitabine | YES | 188 | 6.2 |
| 1612 | Placebo + Gemcitabine | YES | 47 | 1.5 |
| 1613 | Placebo + Gemcitabine | YES | 73 | 2.4 |
| 1614 | Masitinib + Gemcitabine | YES | 312 | 10.3 |
| 1903 | Masitinib + Gemcitabine | YES | 355 | 11.7 |
| 2008 | Masitinib + Gemcitabine | YES | 235 | 7.7 |
| 2009 | Placebo + Gemcitabine | YES | 113 | 3.7 |
| 2403 | Placebo + Gemcitabine | YES | 222 | 7.3 |
| 2703 | Placebo + Gemcitabine | YES | 61 | 2.0 |
| 2704 | Placebo + Gemcitabine | YES | 134 | 4.4 |
| 3107 | Masitinib + Gemcitabine | YES | 483 | 15.9 |
| 3108 | Masitinib + Gemcitabine | YES | 376 | 12.4 |
| 3109 | Masitinib + Gemcitabine | YES | 349 | 11.5 |
| 3110 | Placebo + Gemcitabine | YES | 260 | 8.5 |
| 3111 | Placebo + Gemcitabine | YES | 144 | 4.7 |
| 3112 | Masitinib + Gemcitabine | YES | 112 | 3.7 |

(continued)

| Subject Identifier for the Study | Treatment group | Dead | OS (days) | OS (months) |
|---|---|---|---|---|
| 3308 | Placebo + Gemcitabine | YES | 217 | 7.1 |
| 3309 | Placebo + Gemcitabine | YES | 112 | 3.7 |
| 3406 | Masitinib + Gemcitabine | YES | 104 | 3.4 |
| 3407 | Placebo + Gemcitabine | YES | 171 | 5.6 |
| 3408 | Placebo + Gemcitabine | YES | 350 | 11.5 |
| 3409 | Masitinib + Gemcitabine | YES | 136 | 4.5 |
| 3706 | Placebo + Gemcitabine | NO | 774 | 25.4 |
| 4407 | Placebo + Gemcitabine | YES | 135 | 4.4 |
| 4408 | Masitinib + Gemcitabine | YES | 96 | 3.2 |
| 4409 | Placebo + Gemcitabine | YES | 515 | 16.9 |
| 4410 | Placebo + Gemcitabine | NO | 708 | 23.3 |
| 4411 | Placebo + Gemcitabine | YES | 105 | 3.4 |
| 4412 | Masitinib + Gemcitabine | YES | 194 | 6.4 |
| 4413 | Masitinib + Gemcitabine | YES | 186 | 6.1 |
| 4414 | Placebo + Gemcitabine | YES | 437 | 14.4 |
| 4415 | Placebo + Gemcitabine | YES | 17 | 0.6 |
| 4416 | Masitinib + Gemcitabine | YES | 226 | 7.4 |
| 4503 | Placebo + Gemcitabine | NO | 700 | 23.0 |
| 4702 | Placebo + Gemcitabine | YES | 31 | 1.0 |
| 4703 | Masitinib + Gemcitabine | YES | 141 | 4.6 |
| 4801 | Masitinib + Gemcitabine | YES | 136 | 4.5 |
| 4802 | Masitinib + Gemcitabine | YES | 128 | 4.2 |
| 4803 | Masitinib + Gemcitabine | YES | 258 | 8.5 |
| 4902 | Placebo + Gemcitabine | YES | 161 | 5.3 |
| 4903 | Placebo + Gemcitabine | NO | 602 | 19.8 |
| 5006 | Masitinib + Gemcitabine | YES | 256 | 8.4 |
| 5008 | Placebo + Gemcitabine | YES | 588 | 19.3 |
| 5201 | Placebo + Gemcitabine | YES | 584 | 19.2 |
| 5202 | Placebo + Gemcitabine | YES | 43 | 1.4 |
| 5331 | Placebo + Gemcitabine | YES | 699 | 23.0 |
| 5332 | Masitinib + Gemcitabine | YES | 517 | 17.0 |
| 5333 | Masitinib + Gemcitabine | NO | 128 | 4.2 |
| 5334 | Masitinib + Gemcitabine | YES | 131 | 4.3 |
| 5335 | Masitinib + Gemcitabine | YES | 740 | 24.3 |
| 5336 | Placebo + Gemcitabine | YES | 486 | 16.0 |
| 5337 | Masitinib + Gemcitabine | YES | 265 | 8.7 |
| 5339 | Placebo + Gemcitabine | YES | 65 | 2.1 |
| 5340 | Placebo + Gemcitabine | YES | 356 | 11.7 |

(continued)

| Subject Identifier for the Study | Treatment group | Dead | OS (days) | OS (months) |
|---|---|---|---|---|
| 5341 | Placebo + Gemcitabine | YES | 120 | 3.9 |
| 5342 | Placebo + Gemcitabine | YES | 393 | 12.9 |
| 5343 | Masitinib + Gemcitabine | YES | 107 | 3.5 |
| 5344 | Placebo + Gemcitabine | YES | 667 | 21.9 |
| 5345 | Placebo + Gemcitabine | YES | 251 | 8.2 |
| 5346 | Placebo + Gemcitabine | YES | 163 | 5.4 |
| 5501 | Masitinib + Gemcitabine | YES | 57 | 1.9 |
| 5602 | Masitinib + Gemcitabine | YES | 173 | 5.7 |
| 5702 | Masitinib + Gemcitabine | YES | 115 | 3.8 |
| 5703 | Placebo + Gemcitabine | YES | 261 | 8.6 |
| 5704 | Masitinib + Gemcitabine | NO | 744 | 24.4 |
| 5705 | Masitinib + Gemcitabine | YES | 254 | 8.3 |
| 5901 | Placebo + Gemcitabine | YES | 555 | 18.2 |
| 6201 | Placebo + Gemcitabine | YES | 52 | 1.7 |
| 6301 | Masitinib + Gemcitabine | YES | 341 | 11.2 |
| 6302 | Masitinib + Gemcitabine | YES | 408 | 13.4 |
| 6303 | Placebo + Gemcitabine | YES | 269 | 8.8 |
| 8001 | Placebo + Gemcitabine | YES | 458 | 15.0 |
| 8002 | Masitinib + Gemcitabine | YES | 347 | 11.4 |
| 8003 | Placebo + Gemcitabine | YES | 335 | 11.0 |
| 8106 | Placebo + Gemcitabine | YES | 461 | 15.1 |
| 8107 | Masitinib + Gemcitabine | YES | 373 | 12.3 |
| 8109 | Masitinib + Gemcitabine | YES | 195 | 6.4 |
| 8201 | Masitinib + Gemcitabine | YES | 305 | 10.0 |
| 8501 | Masitinib + Gemcitabine | YES | 216 | 7.1 |
| 8502 | Masitinib + Gemcitabine | YES | 144 | 4.7 |
| 8901 | Placebo + Gemcitabine | YES | 460 | 15.1 |
| 9311 | Masitinib + Gemcitabine | NO | 590 | 19.4 |
| 9312 | Placebo + Gemcitabine | YES | 141 | 4.6 |
| 9508 | Placebo + Gemcitabine | YES | 169 | 5.6 |
| 9509 | Placebo + Gemcitabine | YES | 318 | 10.4 |
| 9901 | Placebo + Gemcitabine | YES | 153 | 5.0 |
| 9903 | Masitinib + Gemcitabine | YES | 181 | 5.9 |
| 10303 | Placebo + Gemcitabine | YES | 131 | 4.3 |
| 10304 | Placebo + Gemcitabine | YES | 234 | 7.7 |
| 10305 | Masitinib + Gemcitabine | YES | 480 | 15.8 |
| 10306 | Masitinib + Gemcitabine | YES | 295 | 9.7 |
| 11001 | Masitinib + Gemcitabine | YES | 57 | 1.9 |

(continued)

| Subject Identifier for the Study | Treatment group | Dead | OS (days) | OS (months) |
|---|---|---|---|---|
| 11205 | Masitinib + Gemcitabine | YES | 168 | 5.5 |
| 11207 | Placebo + Gemcitabine | YES | 231 | 7.6 |

Control of RNA integrity was performed with the 2100 Bioanalyzer (Agilent Technologies, Palo Alto, USA) using Eukaryotic Total RNA 6000 Nano Chip (Agilent Technologies). RNA quantity was controlled using NanoDrop ND-1000 spectrophotometer. Purified RNAs were conserved at -80°C.

3. DGE library construction and tag-to-gene mapping

Twelve Digital Gene Expression (DGE) libraries were constructed from pooled blood RNA samples of patients. For each of the four treatment groups (i.e. Placebo/Gemcitabine **P** or Masitinib + Gemcitabine **M** & dead before month 4, **M4,** or alive after month 15, **M15),** three DGE libraries were constructed using the same pooled blood RNA samples (three technical replicates). The libraries were constructed with Illumina's DGE Tag Profiling kit according to the manufacturer's protocol (version 2.1 B), using 2 $\mu$g of total RNA (equimolar amounts of RNA in the pool between each RNA sample). Sequencing analysis and base calling were carried out using the Illumina Pipeline, and sequence tags were obtained after purity filtering. The platform used was MGX (Montpellier, France). Data from each DGE library were analyzed with BIOTAG software (Skuldtech, Montpellier, France) for tag detection, tag counting and for assessing DGE library quality (Piquemal et al., 2002).

4. Tag annotation and selection

A local database compiling *homo sapiens* sequences and related information from well-annotated sequences of UniGene clusters (Built#232, March 2012, NCBI) was generated. For each sequence of this database, the expected DGE tag (canonical tag) located upstream the 3'-nearest NlaIII restriction site (CATG) of the sequence (R1), as well as putative tags located in inner positions (labeled as R2, R3 and R4 starting from the 3' end of the transcript), were extracted (Piquemal et al., 2002). Experimental tags obtained from DGE libraries were matched and annotated (exact matches for the 17bp) using this collection of virtual tags. Firstly, a correspondence for each experimental tag with the virtual canonical tags (R1) was looked for. Then, unmatched experimental tags with the R2 tags, then with R3, and R4 were annotated.

The analyses of the DGE experiments were carried out using edgeR Method (version 2.6.9, Bioconductor). The analyzed genes were selected according to (1) mathematic filters with the highest differential Fold Change (>1.5), FDR (False Discovery Rate) adjusted p-value criterion (<10%) based on the type I ($\alpha$=5%) error reported in *General considerations* and (2) biologic filters with involvement of targeted genes in specific processes and known metabolic pathways.

5. cDNA synthesis for Real-Time PCR

Reverse transcriptions were carried out for each of the 120 RNA in 20 $\mu$l final reaction volume with 300ng of total RNA using 200 units of SuperScript II enzyme (M-MLV RT Type, Invitrogen) and 250ng of random primers according to manufacturer's instructions (25°C 10 min, 42°C 50 min, 70°C 15 min), the same day with the same pipettor set and the same manipulator.

6. Real-Time PCR

The validation of targeted genes was carried out on Real-Time PCR (qPCR) platform from Roche Diagnostics.
The qPCR experiments were carried out using LightCycler® 1536 DNA Green Master Kit and RealTime ready DNA Probes Master Kit (Roche Diagnostics) on Roche Diagnostics LightCycler1536® qPCR apparatus according to manufacturer's instructions.
For Sybr Green assays, the reaction mixture was prepared in a final volume of 2 $\mu$l as follows: 0,4 $\mu$l of LightCycler 1536 DNA Green Master 5X (Roche), 0,1$\mu$l of Bright Green 20X (Roche), 0,1 $\mu$l of Setup Control 20X (Roche), 0,04 $\mu$l of 50 $\mu$M primers couple (Eurogentec), 0,36$\mu$L of DNAse RNAse free water and 1 $\mu$l of cDNA matrix (1/50 final dilution). For probes assays, the reaction mixture was prepared in a final volume of 2 $\mu$l as follows: 0,4$\mu$l of Real Time Ready DNA Probe Master 5X (Roche), 0,1 $\mu$l of Control Setup 20X, 0,1 $\mu$l of 4 $\mu$M Forward primer (Eurogentec),

0,1μL of 4μM Reverse primer (Eurogentec), 0,1μL of 4μM FAM/TAMRA Probe (Eurogentec), 0,2μl of DNAse RNAse free water and 1 μl of cDNA matrix (1/50 final dilution). All pipetting steps were carried out with Agilent Bravo Automated Liquid Handling Platform.

PCR program consists in a first pre-incubation step at 95°C for 1 min following by 50 PCR cycles (95°C for 2 sec, 60°C for 30 sec). To discriminate specific from nonspecific products and primer dimers, a melting curve was obtained by gradual increase in temperature from 60 to 95°C.

Table: Real-Time PCR primers of the 7 Biomarkers plus the 2 reference genes (* housekeeping genes)

| Biomarker | Primer Forward | Primer Reverse |
|---|---|---|
| ING5 | TCCCTTTGTAAAGGAAAGATAATG | TCCATAATCTATAACATGATCCAAAAA |
| S100A6 | CCTCAGCCATGGC(LNA-base)AC(LNA-base)G | ATCAGCTCCTTCAGCTCCTTC |
| RUNDC1 | TCACCTTCCCTGTGAAACAA | CCAAAGCTGCAGATCTCTAAGG |
| ANKRD5 | GTTTGCTTTGTGGCATGTG | CATGTTTGTCTGGGATGTGG |
| ABCC1 | CCAGTGGGGATCGGACAGA | AGGGGATCATCGAAGAGGTAAAT |
| CD8A | GGGTTGTACTATGCTGTTATGAGTG | TGTTTACCCATTAAAGAGGATGC |
| FGFR3 | ACCCAGTGCAGAATGTAAGTG | GTGATGTCCCGTGGTGCAA |
| B2M* | GCTCAGTAAAGACACAACCATCC | CATCTGTGGATTCAGCAAACC |
| GAPDH* | ATGGGGAAGGTGAAGGTCG | GGGGTCATTGATGGCAACAATA |

The qPCR data were analyzed using the Delta.Ct ($\Delta$Ct) method (Livak and Schmittgen, 2001). The $\Delta$Ct values were determined for all target genes by subtracting the Ct values from the mean of the Ct values of the two reference genes (housekeeping). The 2 housekeeping genes are B2M (NM_009735, *Mus musculus* beta-2 microglobulin, mRNA) and GAPDH (NM_002046, glyceraldehyde-3-phosphate dehydrogenase, transcript variant 1, mRNA + NM_001256799 *Homo sapiens* glyceraldehyde-3-phosphate dehydrogenase, transcript variant 2, mRNA).

7. Results

Using the Digital Gene Expression (DGE) method, the transcriptomic profiles of total blood of patients was carried out and 169 genes have been selected with edgeR Method. The analyzed genes have been selected according to (1) mathematic filters with the highest differential Fold Change (>1.5), FDR adjusted p-value criterion (<10%) based on the type I ($\alpha$=5%) error and (2) biological filters with involvement of targeted genes in specific processes and known metabolic pathways.

In a real time PCR assay, a positive reaction is detected by accumulation of a fluorescent signal. The Ct (cycle threshold) is defined as the number of cycles required for the fluorescent signal to cross the threshold (i.e. exceeds background level). Ct values are inversely proportional to the amount of target nucleic acid in the sample (i.e. the lower the Ct value, the greater the amount of target nucleic acid in the sample).

The clinical phase III study (from AB Science, Id. AB07012) provided samples for an ancillary pharmacogenomic study. RNA blood samples were taken from 120 patients before any treatment and they were analyzed via RT-PCR (reverse transcription polymerase chain reaction). A "genetic pro-metastatic fingerprint" was isolated, present in 65% of patients, which was highly predictive for overall survival, and furthermore, interacted with the treatment type.

In particular, placebo/gemcitabine-treated patients with the "genetic pro-metastatic fingerprint" had the lowest median overall survival (OS) (5.0 months) whereas patients with this "genetic pro-metastatic fingerprint" treated with masitinib plus gemcitabine had a median OS of 11 months, meaning that OS was increased by 6 months (*p*-value = 0.000038).

Among the 169 genes, ING5, RUNDC1, ABCC1, FGFR3, S100A6, ANKRD55, and CD8A genes were selected by the inventors, in agreement with the multi-factorial nature of this indication.

| Name | Description | Gene identifiant Sequence Id.(Ensemble) | Example of mRNA sequences Sequence Id. (Genbank) |
|---|---|---|---|
| ABCC11 | ATP-binding cassette, sub-family C (CFTR/MRP), member 1 | ENSG00000103222 | NM_004996 NM_019862 NM_019898 NM_019899 NM_019900 NM_019901 NM_019902 |
| ANKRD55 | ankyrin repeat domain 55 | ENSG00000164512 | NM_001039935 NM_024669 |
| CD8A | CD8a molecule | ENSG00000153563 | NM_001145873 NM_001768 NM_171827 NR_027353 |
| FGFR3 | Fibroblast growth factors receptor 3 | ENSG00000068078 | NM_000142 NM_022965 NM_001163213 |
| ING5 | inhibitor of growth family, member 5 | ENSG00000168395 | NM_032329 |
| RUNDC1 | RUN domain containing 1 | ENSG00000198863 | NM_173079 |
| S100A6 | S100 calcium binding protein A6 | ENSG00000197956 | NM_014624 |
| GAPDH | glyceraldehyde-3-phosphate dehydrogenase | ENSG00000111640 | NM_002046 NM_001256799 |
| B2M | beta-2 microglobulin | ENSG00000166710 | NM_009735 |

Up to today, no results of treatment of a genetic population in pancreatic cancer patients have been reported. Therefore, the identification of a genetic fingerprint described here opens a new avenue to personalized therapy in this indication.

The genetic pro-metastatic fingerprint, based on a specific Delta.Ct ($\Delta$Ct) value, can be routinely determined via RT-PCR (reverse transcription polymerase chain reaction) from RNA blood samples. The $\Delta$Ct value illustrating the expression level of a given gene in a given patient is obtained from the amplification by RT-PCR of a given gene and after individual normalization with respect to genes of reference (B2M, GAPDH). The lower the $\Delta$Ct value, the higher the expression level of the gene.

Patients having long-term survival and thus eligible for Gemcitabine + Masitinib treatment present a specific expression pattern in at least one of the 4 following gene combinations:

- Combination 1: ING5 and RUNDC1 for which the $\Delta$Ct value of the patient is higher than the median $\Delta$Ct of all patients tested (i.e. higher than 10.705 for ING5 and 3.756 for RUNDC1);
- Combination 2: ABCC1 for which the $\Delta$Ct value of the patient is higher than the median $\Delta$Ct (4.109) and FGFR3 for which the $\Delta$Ct value of the patient is higher than the first quartile $\Delta$Ct (13.082),
- Combination 3: ING5 and S100A6 for which the $\Delta$Ct value of the patient is higher than the median $\Delta$Ct (10.705 for ING5 and 4.472 for S100A6),
- Combination 4: ANKRD55 for which the $\Delta$Ct value of the patient is higher than the median $\Delta$Ct (6.258) and CD8A for which the $\Delta$Ct value of the patient is higher than the first quartile $\Delta$Ct (5.303).

## Claims

1.    An *in vitro* method for determining the prognosis of pancreatic cancer in a patient, comprising the following steps:

a) measuring the expression level of at least two genes selected from the group consisting in: ING5, RUNDC1, ABCC1, FGFR3, S100A6, ANKRD55, and CD8A or homologous genes, in a blood sample of said patient,
b) predicting the outcome of the pancreatic cancer in said patient.

2. The method according to claim 1, wherein the expression level of seven genes is measured.

3. The method according to any one of claims 1 and 2, wherein a lower expression level of at least two of the ING5, RUNDC1, ABCC1, FGFR3, S100A6, ANKRD55, and CD8A genes indicates a long-term survival of said patient.

4. The method according to any one of claims 1 and 2, wherein a higher expression level of all of the ING5, RUNDC1, ABCC1, FGFR3, S100A6, ANKRD55, and CD8A genes indicates a short-term survival of said patient.

5. The method according to any one of claims 1 to 4, wherein said blood sample is a peripheral whole blood sample.

6. The method according to any one of claims 1 to 5, wherein the expression level of a gene is measured as the level of the RNA transcript or the cDNA of said gene.

7. The method according to any one of claims 1 to 5, wherein the expression level of a gene is measured as the level of the protein of said gene.

8. The method according to any one of claims 1 to 6, wherein the expression level of a gene is measured by a real time quantitative PCR performed on the RNA transcript or the cDNA of said gene, to determine the cycle threshold (Ct) value, said value being normalized with respect to the expression level of a housekeeping gene to give a value $\Delta$Ct.

9. The method according to claim 8, wherein the $\Delta$Ct is based on the expression level of two housekeeping genes, preferably, genes B2M and GAPDH.

10. The method according to claim 9, wherein a patient having a $\Delta$Ct value for ING5 and RUNDC1, that is higher than the median $\Delta$Ct for said genes, has a long-term survival.

11. The method according to claim 9, wherein a patient having a $\Delta$Ct value for ABCC1 that is higher than the median $\Delta$Ct for said gene and a $\Delta$Ct value for FGFR3 that is higher than the 1$^{st}$ quartile $\Delta$Ct for said gene, has a long-term survival.

12. The method according to claim 9, wherein a patient having a $\Delta$Ct value for ING5 and S100A6, that is higher than the median $\Delta$Ct for said genes, has a long-term survival.

13. The method according to claim 9, wherein a patient having a $\Delta$Ct value for ANKRD55 that is higher than the median $\Delta$Ct for said gene and a $\Delta$Ct value for CD8A that is higher than the 1$^{st}$ quartile $\Delta$Ct for said gene, has a long-term survival.

14. The method according to any one of claims 1 to 13, wherein the pancreatic cancer prognosis is the prognosis further to a pancreatic cancer treatment, which is preferably a gemcitabine-based treatment.

15. A kit for determining the prognosis of pancreatic cancer in a patient, comprising means for detecting the level of expression of at least two genes selected from the group consisting in ING5, RUNDC1, ABCC1, FGFR3, S100A6, ANKRD55, and CD8A.

16. The use of at least two genes selected in the group consisting in ING5, RUNDC1, ABCC1, FGFR3, S100A6, ANKRD55 and CD8A for the prognosis of pancreatic cancer in a patient.

Europäisches
Patentamt

European
Patent Office

Office européen
des brevets

# EUROPEAN SEARCH REPORT

Application Number

EP 12 30 6209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AGILENT TECHNOLOGIES: "Agilent SurePrint G3 Human Catalog CGH Microarrays", INTERNET CITATION, 8 January 2009 (2009-01-08), pages 1-8, XP002660065, Retrieved from the Internet: URL:http://www.chem.agilent.com/Library/brochures/5990-3368en_lo.pdf [retrieved on 2011-09-26] * the whole document * | 15 | INV. C12Q1/68 |
| X | AFFYMETRIX: "GeneChip TM Human Genome U133 Arrays", DATASHEET AFFYMETRIX,, 1 January 2003 (2003-01-01), pages 1-8, XP007921348, * the whole document * | 15 | |
| X | Illumina: "Whole-genome expression analysis using the Sentrix Human-6 and HumanRef-8 expression beadchips", , 28 June 2005 (2005-06-28), pages 1-8, XP055005291, Retrieved from the Internet: URL:http://www.illumina.com/Documents/products/techbulletins/techbulletin_whole_genome_expression.pdf [retrieved on 2011-08-19] * the whole document * | 15 | TECHNICAL FIELDS SEARCHED (IPC) C12Q |

-/--

The present search report has been drawn up for all claims

| Place of search | Date of completion of the search | Examiner |
|---|---|---|
| Munich | 7 March 2013 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 12 30 6209

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GANG ZHOU ET AL: "Detection and Clinical Significance of CD44v6 and Integrin-Î1 in Pancreatic Cancer Patients using a Triplex Real-Time RT-PCR Assay", APPLIED BIOCHEMISTRY AND BIOTECHNOLOGY ; PART A: ENZYME ENGINEERING AND BIOTECHNOLOGY, HUMANA PRESS INC, NEW YORK, vol. 167, no. 8, 14 June 2012 (2012-06-14) , pages 2257-2268, XP035098339, ISSN: 1559-0291, DOI: 10.1007/S12010-012-9752-2 | 1,3,5-9, 14 | |
| A | * the whole document * | 2,4, 10-13 | |
| Y | S. BALASENTHIL ET AL: "A Migration Signature and Plasma Biomarker Panel for Pancreatic Adenocarcinoma", CANCER PREVENTION RESEARCH, vol. 4, no. 1, 1 January 2011 (2011-01-01) , pages 137-149, XP055055351, ISSN: 1940-6207, DOI: 10.1158/1940-6207.CAPR-10-0025 | 1,3,5-9, 14 | TECHNICAL FIELDS SEARCHED (IPC) |
| A | * the whole document * | 2,4, 10-13 | |
| Y | JÖRG KÖNIG ET AL: "Expression and localization of human multidrug resistance protein (ABCC) family members in pancreatic carcinoma", INTERNATIONAL JOURNAL OF CANCER, vol. 115, no. 3, 20 June 2005 (2005-06-20) , pages 359-367, XP055055620, ISSN: 0020-7136, DOI: 10.1002/ijc.20831 * the whole document * | 1,3,5-9, 14 | |

-/--

The present search report has been drawn up for all claims

| Place of search | Date of completion of the search | Examiner |
|---|---|---|
| Munich | 7 March 2013 | Schmitt, Anja |

EPO FORM 1503 03.82 (P04C01)

Europäisches
Patentamt

European
Patent Office

Office européen
des brevets

# EUROPEAN SEARCH REPORT

Application Number

EP 12 30 6209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | K. OHUCHIDA ET AL: "S100A6 Is Increased in a Stepwise Manner during Pancreatic Carcinogenesis: Clinical Value of Expression Analysis in 98 Pancreatic Juice Samples", CANCER EPIDEMIOLOGY, BIOMARKERS & PREVENTION, vol. 16, no. 4, 1 April 2007 (2007-04-01), pages 649-654, XP055055642, ISSN: 1055-9965, DOI: 10.1158/1055-9965.EPI-06-0157 * the whole document * | 1,3,5-9, 14 | |
| A | LI Z ET AL: "The myeloma-associated oncogene fibroblast growth factor receptor 3 is transforming in hematopoietic cells", BLOOD, AMERICAN SOCIETY OF HEMATOLOGY, US, vol. 97, no. 8, 15 April 2001 (2001-04-15) , pages 2413-2419, XP002452623, ISSN: 0006-4971, DOI: 10.1182/BLOOD.V97.8.2413 * the whole document * | 1-15 | |
| A | K. OHUCHIDA: "The Role of S100A6 in Pancreatic Cancer Development and Its Clinical Implication as a Diagnostic Marker and Therapeutic Target", CLINICAL CANCER RESEARCH, vol. 11, no. 21, 1 November 2005 (2005-11-01), pages 7785-7793, XP055055288, ISSN: 1078-0432, DOI: 10.1158/1078-0432.CCR-05-0714 * the whole document * | 1-15 | |

TECHNICAL FIELDS SEARCHED (IPC)

-/--

The present search report has been drawn up for all claims

| Place of search | Date of completion of the search | Examiner |
|---|---|---|
| Munich | 7 March 2013 | Schmitt, Anja |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**Europäisches Patentamt**

**European Patent Office**

**Office européen des brevets**

# EUROPEAN SEARCH REPORT

**Application Number**

EP 12 30 6209

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WALZAK ET AL: "Expression profiles of mRNA transcript variants encoding the human inhibitor of growth tumor suppressor gene family in normal and neoplastic tissues", EXPERIMENTAL CELL RESEARCH, ACADEMIC PRESS, US, vol. 314, no. 2, 7 December 2007 (2007-12-07), pages 273-285, XP022382695, ISSN: 0014-4827, DOI: 10.1016/J.YEXCR.2007.07.029 * the whole document * ----- | 1-15 | |
| A | SEYED MEHDI JAFARNEJAD ET AL: "Regulation of p53 by ING family members in suppression of tumor initiation and progression", CANCER AND METASTASIS REVIEWS, KLUWER ACADEMIC PUBLISHERS, DO, vol. 31, no. 1 - 2, 18 November 2011 (2011-11-18), pages 55-73, XP035054925, ISSN: 1573-7233, DOI: 10.1007/S10555-011-9329-5 * the whole document * ----- | 1-15 | **TECHNICAL FIELDS SEARCHED (IPC)** |

The present search report has been drawn up for all claims

| Place of search | Date of completion of the search | Examiner |
|---|---|---|
| Munich | 7 March 2013 | Schmitt, Anja |

EPO FORM 1503 03.82 (P04C01)